# EUROPEAN PATENT SPECIFICATION

(11) **EP 3 815 651 B1**
(45) Date of publication and mention of the grant of the patent: **06.03.2024**
(21) Application number: 19827232.0
(22) Date of filing: 21.06.2019
(51) Int. Cl.: A61F 2/958, A61F 2/95

(54) **MEDICAL DEVICE AND PREPARATION METHOD THEREFOR**
MEDIZINPRODUKT UND VERFAHREN ZU SEINER HERSTELLUNG
DISPOSITIF MÉDICAL ET SON PROCÉDÉ DE PRÉPARATION

(30) Priority: 29.06.2018 CN 201810712962
(43) Date of publication of application: 05.05.2021
(73) Proprietor: Shanghai MicroPort Medical (Group) Co., Ltd., Shanghai 201203 (CN)
(72) Inventor: LI, Dan, Shanghai 201203 (CN); LIU, Wei, Shanghai 201203 (CN); WANG, Xueqin, Shanghai 201203 (CN); QI, Fan, Shanghai 201203 (CN); YAO, Yingzhong, Shanghai 201203 (CN); LUO, Qiyi, Shanghai 201203 (CN)
(74) Representative: Marks & Clerk LLP
(86) International application number: PCT/CN2019/092359
(87) International publication number: WO 2020/001386

(56) References cited:
- WO-A1-2008/079557
- CN-A- 102 836 022
- CN-U- 208 910 614
- US-A1- 2002 138 128
- US-A1- 2005 151 304
- US-A1- 2008 154 352
- US-A1- 2017 348 123

## Description

### Technical Field

The present invention relates to the field of medical devices and, in particular, to a medical device and a method of manufacturing it.

### Background

A polymer stent product, consisting of a polymer stent, a drug coating and a balloon delivery system, is suitable for use in treating an ischemic heart disease caused by a primary coronary artery dissection and improving the diameter of the coronary artery lumen.

In many therapeutic applications, a polymer stent is required to stay in the patient's body for a limited period of time in order to fulfill its intended functionality. Since polymer stents are inferior in terms of mechanical properties compared to metal stents, they are generally made thicker and wider in order to provide sufficient strength that would be required by the walls of blood vessels where they are deployed.

Relatively speaking, a polymer stent has a larger diameter in a circumferentially crimped configuration and exhibits a greater tendency of radial resilience prior to use. Moreover, when the polymer stent is used in a surgical procedure, it is crimped and loaded onto a traditional balloon solely by crimping forces. For this reason, during its delivery toward a distant target site in the patient body through a more or less clotted, curved or tortuous blood vessel portion, the relatively large radial diameter and resilience will make it easier to dislodge, or bring damage to the wall of the blood vessel, or be subject to a "flaring" effect resulting from the bifurcating and flaring at its one end. All of these will lead to an increase in surgical risk. US 2008/154352 A1 discloses a method of manufacturing a medical device, i.e. a stent crimped on a balloon and a medical device comprising a stent and a balloon wherein the medical device is manufactured by such a method,

### Summary of the Invention

In view of the above, the present invention provides a medical device and a method of manufacturing it, which allow effective formation of a protrusion on a balloon arrangement while not adversely affecting the performance of the balloon arrangement during use and increasing dislodgement resistance of a stent. As a result, during delivery of the stent, it can be prevented from dislodging from the balloon arrangement.

According to one aspect of the present invention, there is provided a method of manufacturing a medical device, including:
providing a preform of a balloon arrangement in a folded configuration;
crimping a stent onto the preform;
disposing a preforming mold over the preform on which the stent is crimped so that the preform on which the stent is crimped is housed in a molding chamber of the preforming mold; and
heating the preform in the molding chamber and filling a fluid in the preform so that a protrusion is formed and shaped on the preform in the molding chamber, thereby obtaining a molded structure of the balloon arrangement.

Preferably, in this method, protrusions are formed at both ends of the molded structure, and the stent is crimped on the molded structure and located between the protrusions at both ends of the molded structure.

According to the invention, in this method, the protrusion(s) has a cross section of an inverted L shape along an axial direction of the molded structure.

Preferably, in this method, during the obtainment of the molded structure, a surface of the protrusion(s) is fitted against an outer surface of the crimped stent so that the protrusion(s) wraps the stent.

Preferably, in this method, after each protrusion has been formed and shaped on the preform in the molding chamber, the heating of the preforming mold is stopped, with the filling of the fluid into the preform being continued until the preforming mold has cooled.

Preferably, in this method, the preform is heated at a temperature of from 40 °C to 80 °C, and wherein the fluid is a compressed gas supplied at a pressure of from 2.0 atm to 10.0 atm.

According to another aspect of the present invention, there is provided a medical device including a stent and a balloon arrangement configured to release the stent in an expanded configuration thereof. The medical device is manufactured by the method as defined above, and the balloon arrangement includes a molded structure with a protrusion. The stent is crimped on the balloon arrangement and located on a side where the protrusion is formed.

Preferably, in this medical device, the protrusion is circumferentially continuous, or the protrusion includes a plurality of portions, the plurality of portions spaced apart from one another along a circumferential direction of the molded structure.

Preferably, in this medical device, an axial clearance between the protrusion and the stent is smaller than or equal to 5 mm.

Preferably, in this medical device, the protrusion defines an opening that is open toward the molded structure and configured to accommodate an end portion of the stent.

Preferably, in this medical device, a surface of the protrusion is fitted against an outer surface of the crimped stent so that the protrusion wraps at least part of the stent.

Preferably, in this medical device, 0 to 1.0 ring of the stent in an axial direction is wrapped by the protrusion.

According to the invention, in this medical device, the protrusion has a cross section of an inverted L shape along an axial direction of the molded structure.

The medical device and method according to the present invention offer the following advantages:
First, a preform of an existing balloon arrangement in a folded configuration can be reshaped in a preforming mold at a predetermined temperature and fluid pressure into a molded structure, which is in a folded configuration and has a protrusion at at least one end. Since the protrusion is formed on the folded balloon arrangement, its use will not be adversely affected by a balloon folding process. This ensures that the protrusion will not shrink or disappear due to folding of the balloon arrangement. Thus, this approach can be used to effectively produce a balloon arrangement with protrusion(s). In particular, any protrusion created in this manner only exists when the balloon arrangement is in a folded configuration and will disappear when the balloon arrangement is inflated and expanded. Therefore, it will not impose any adverse impact on the performance of the balloon arrangement.

Second, when a stent is crimped onto the balloon arrangement in such a manner that it is located on the side where the protrusion is present, the protrusion can axially restrain the stent and thus prevent it from dislodging from the balloon, resulting in an increase in dislodgement resistance of the stent and a reduction in surgical risk. Preferably, in addition to restraining the stent axially, the stent may be, for example, inverted L-shaped to further restrain the stent radially, resulting in an additional increase in the stent's dislodgement resistance. More preferably, such protrusions may be formed at both ends of the balloon arrangement. In this case, the stent can be crimped onto a section of the balloon arrangement between the protrusions. This can also result in an additional increase in dislodgement resistance of the stent.

Third, when the protrusion is formed beside a distal end of the stent, during delivery of the stent in a blood vessel, the protrusion will come into contact with the wall of the blood vessel prior to the distal end of the stent. This can reduce a direct contact area between the stent end and the blood vessel wall, and since the balloon arrangement is made of a material that is softer than the material from which a stem of the stent is fabricated, possible damage brought by the more rigid stent stem to the blood vessel wall can be reduced, and it is made easier to prevent bifurcation of the stent. The inverted L-shape of the protrusion, especially such a shape wrapping a distal end portion of the stent, will create a soft transition area around the stent end, which can effectively reduce damage to the blood vessel wall during delivery. Further, wrapping the stent end with the balloon arrangement can avoid direct contact of the stent end with the blood vessel wall. So, when the blood vessel is tortuous and calcified, the stent end will not bifurcate into a flare shape.

### Brief Description of The Drawings

The accompanying drawings are provided for a better understanding of the present invention and do not limit it in any way. In these figures:
Fig. 1a is a schematic illustration of a balloon arrangement according to a first embodiment of the present invention;
Fig. 1b is a schematic illustration of the balloon arrangement with a stent crimped thereon according to the first embodiment of the present invention;
Fig. 2a is a schematic illustration of a balloon arrangement according to a second embodiment of the present invention;
Fig. 2b is a schematic illustration of the balloon arrangement with a stent crimped thereon according to the second embodiment of the present invention;
Fig. 3 is a schematic illustration of the balloon arrangement with a stent crimped thereon according to a third embodiment of the present invention;
Fig. 4a is an axial schematic illustration of a preform provided in the manufacturing of a balloon arrangement in accordance with an embodiment of the present invention;
Fig. 4b shows an end view of the preform provided in the manufacturing of a balloon arrangement, which has been folded, in accordance with an embodiment of the present invention;
Fig. 5a is an exploded view of a preforming mold according to an embodiment of the present invention;
Fig. 5b is a schematic diagram of a fixture according to an embodiment of the present invention;
Fig. 5c is a schematic diagram of a fixture disposed over a preforming mold according to an embodiment of the present invention;
Fig. 5d is a diagram illustrating how a balloon arrangement is manufactured in accordance with an embodiment of the present invention;
Fig. 6 is a schematic diagram of a preform with a stent crimped thereon in advance during the manufacturing of a balloon arrangement in accordance with another embodiment of the present invention;
Fig. 7 is an axial cross-sectional view of a preforming mold according to another embodiment of the present invention;
Fig. 8 is a diagram illustrating how a balloon arrangement is manufactured in accordance with another embodiment of the present invention;
Fig. 9 is a schematic diagram of a preforming mold used in the manufacturing of a balloon arrangement in accordance with another embodiment of the present invention;
Fig. 10 is a diagram illustrating how a balloon arrangement is manufactured in accordance with another embodiment of the present invention;
Fig. 11 is a diagram illustrating how a balloon arrangement is manufactured in accordance with another embodiment of the present invention;
Fig. 12 is a diagram illustrating how a balloon arrangement is manufactured in accordance with another embodiment of the present invention; and
Fig. 13 is a diagram illustrating how a balloon arrangement is manufactured in accordance with another embodiment of the present invention;

In these figures,
10, 20 and 30-balloon arrangement; 101-preform; 11, 21 and 31-protrusions; 100-stent; 410, 510, 610 and 710-preforming mold; 411-first molding body; 413-first molding cavity; 412-second molding body; 414-second molding cavity; 420, 520, 620 and 720-fixture; 511 and 716-molding chamber; 611 and 711-first shaping mold; 612 and 712-second shaping mold; 613 and 713-first confining mold; 614 and 714-second confining mold; 6131-first flap; 6132-second flap; 6133-third flap; 6141-fourth flap; 6142-fifth flap; 6143-sixth flap; 615 and 715-confining cavity; 7111-first diameter; 7112-second diameter; 7121-third diameter; 7122-fourth diameter.

### Detailed Description of Preferred Embodiments

The above and other objectives, advantages and features of the invention will be more readily apparent from the following detailed description made in conjunction with the accompanying drawings. Note that the figures are provided in a very simplified form not necessarily presented to scale, with the only intention of facilitating convenience and clarity in explaining the embodiments disclosed hereinafter.

As used herein and in the appended claims, the singular terms "a", "an" and "the" include plural referents unless the context clearly dictates otherwise. As used herein and in the appended claims, the term "or" is generally employed in the sense including "and/or", unless the context clearly dictates otherwise.

As used herein and in the appended claims, the term "internal" generally refers to a location closer to an axis of a component, while the term "external" generally refers to a location farther away from an axis of a component. The term "axial" generally refers to a direction parallel to a center axis of a component, while the term "radial" generally refers to a direction perpendicular to a center axis of a component. The term "circumferential" generally refers to a direction about an axis of a balloon arrangement. The term "length" generally refers to a distance along the direction of an axis of a stent. The term "height" generally refers to a distance from an outer surface of a folded balloon along the radial direction of a balloon arrangement. The term "folded configuration" generally refers to a non-inflated or contracted configuration of a balloon. Examples of the folded configuration may include, but are not limited to, single-, bi- and tri- fold configurations.

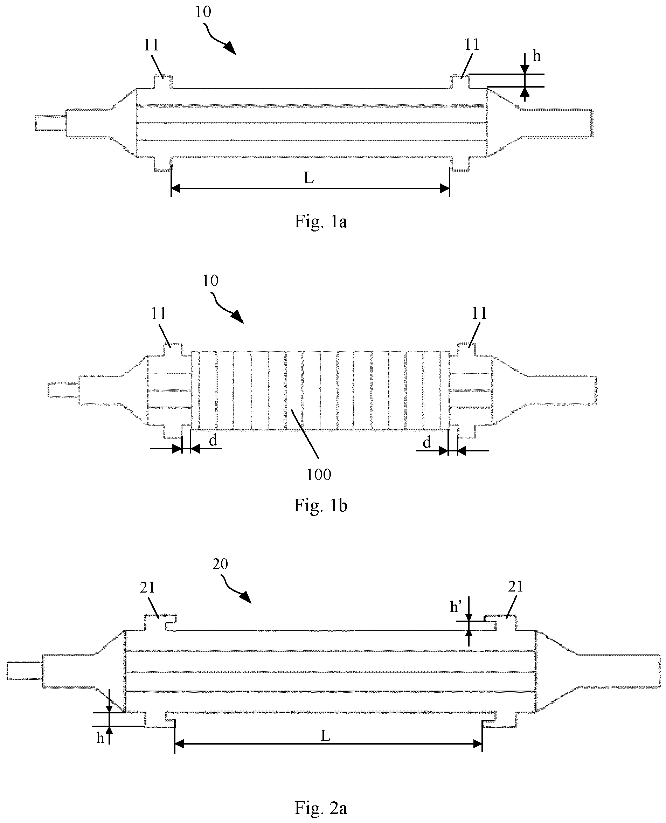

As shown in Figs. 1a and 1b, in a first embodiment of the present invention, there is provided a balloon arrangement 10. The balloon arrangement 10 includes a body (i.e., a molded structure) and protrusions 11 arranged at both ends of the body. A stent 100 can be crimped between the protrusions 11 at the respective ends, each protrusion 11 projects to a height that is at least higher than that of an inner surface of the crimped stent 100 so as to limit the stent 100 at least in the axial direction of the balloon arrangement 10, thereby preventing the stent 100 from dislodging from the balloon arrangement 10 and providing the stent with improved dislodgement resistance. Moreover, it is to be noted that the height of each protrusion 11 may be further higher than that of an outer surface of the crimped stent 100. However, in order to avoid an unnecessarily large diameter of the balloon arrangement 10, the height of each protrusion 11 is preferably smaller than that of the outer surface of the crimped stent 100. In other words, a surface of each protrusion 11 is positioned radially external to the inner surface of the crimped stent 100 (i.e., a radially outermost surface portion of each protrusion 11 is external to the inner surface of the stent 100) and internal to the outer surface of the stent 100. Here, the balloon arrangement 10 is in a folded configuration and surrounded by the crimped stent 100 that is expandable under the action of the balloon arrangement 10. The crimped stent 100 is in a radially inward contracted configuration. In addition, the height of the inner surface of the crimped stent 100 refers to a distance from the inner surface to an outer surface of the body in a radial direction. The height of each protrusion 11 refers to a height of the body's outer surface bulging outward in the radial direction.

In the first embodiment, the protrusions 11 at both ends of the body are spaced from each other by a distance L that is greater than a length of the stent 100 so that the stent 100 can be crimped between the protrusions 11. Preferably, each protrusion 11 is axially spaced from the stent 100 by a clearance d ranging from 0 mm to 5.0 mm. More preferably, the protrusions 11 are in mirror symmetry with respect to an axis of symmetry of the stent 100. Each protrusion 11 may have a rectangular cross section along an axis of the balloon arrangement 10. The protrusions 11 are integral with the body of the balloon arrangement 10. In this embodiment, the height h of each protrusion 11 ranges from 50 µm to 200 µm, with from 50 µm to 150 µm being more preferred.

Further, each protrusion 11 is not limited to a circumferentially (around the balloon) continuous structure, because it may also consist of several portions that may be identical to one another. Each of these portions may be considered as an independent protrusion 11, and these protrusions 11 may be spaced from one another circumferentially around the body. For example, two protrusions may be distributed in symmetry, or three protrusions may be distributed in an angularly equidistant manner in which every two of them are spaced from each other by 120°. Therefore, at each end of the balloon arrangement 10, there may be arranged either multiple protrusions 11 circumferentially spaced from one another, or a single protrusion 11 that is circumferentially continuous. The number of the protrusions, as well as how they are arranged, may be determined as actually needed.

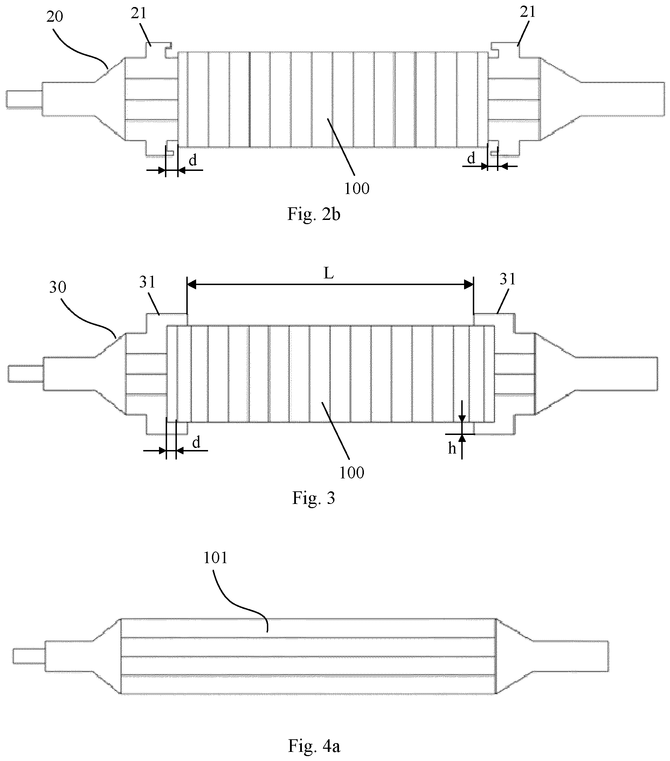

As shown in Figs. 2a and 2b, in a second embodiment of the present invention, there is provided another balloon arrangement 20. The balloon arrangement 20 also includes a body (i.e., a molded structure) and protrusions 21 arranged at both ends of the body. A stent 100 can be crimped between the protrusions 11 at the respective ends, and each protrusion 21 defines an opening that is open toward the body (of the balloon arrangement 20) and adapted to receive a respective end of the stent 100. Moreover, a minimum distance L between the protrusions 21 at the respective ends is greater than a length of the stent 100, and a surface of each protrusion 21 closer to the body is higher than an outer surface of the crimped stent 100 so that the stent 100 can be received in the opening. In this way, when the stent 100 is crimped onto the body, it can be limited by the protrusions 21 both axially and radially, resulting in an additional increase in the stent's dislodgement resistance. In this embodiment, each protrusion 21 may be inverted L-shaped (in the axial section plane). Likewise, each protrusion 11 is not limited to a circumferentially continuous structure, because it may also consist of several circumferentially portions spaced one from another. Additionally, each protrusion 21 has a height that should not be too large. Optionally, the opening of each protrusion 21 may have a height h' that is greater than or equal to a thickness of the crimped stent, and a total height h of each protrusion 21 may be greater than a height of the outer surface of the crimped stent. Optionally, in this embodiment, the total height h of each protrusion 21 may range from 50 µm to 200 µm, with from 50 µm to 150 µm being more preferred. In addition, each protrusion 21 may be axially spaced from the stent 100 by a clearance d ranging from 0 mm to 5.0 mm. More preferably, the protrusions 11 are in mirror symmetry with respect to an axis of symmetry of the stent 100.

As shown in Fig. 3, there is also provided a balloon arrangement 30 in a third embodiment of the present invention, the balloon arrangement 30 is similar to that of the second embodiment, except that a stent 100 is wrapped by protrusions 31 arranged on both ends of the balloon arrangement 30. In order for improved dislodgement resistance of the stent to be achieved, each protrusion 31 preferably covers an axial length of the stent that is equal to one ring width. Here, the "ring width" refers to a maximum axial width of each of annular elements that make up the stent by connecting to one another. When covering the stent, each protrusion 31 preferably has a surface brought into contact with an outer surface of the crimped stent 100 (i.e., the height of an opening defined by the protrusion is equal to a thickness of the crimped stent). To this end, each protrusion 31 may be also inverted L-shaped (in the axial section plane). Likewise, each protrusion 11 may be either a circumferentially continuous structure or may consist of several circumferentially protrusion 31 spaced one from another.

Further, in the third embodiment, a minimum distance L between the protrusions 31 at the respective ends is smaller than a length of the stent 100. More preferably, the protrusions 31 are in mirror symmetry with respect to an axis of symmetry of the stent 100. Optionally, in this embodiment, a total height h of each protrusion 31 may range from 50 µm to 200 µm, with from 50 µm to 150 µm being more preferred.

It is to be noted that the present invention is not limited to arranging protrusions at both ends of the balloon arrangement. Alternatively, it is also possible to arrange only one protrusion at one end thereof. In this case, a crimped stent may be disposed on the balloon arrangement so that it is closer to the end where the protrusion is arranged. The only one protrusion can also limit the stent during delivery, thereby preventing the stent from dislodging from the balloon arrangement. With similarity to the embodiments with protrusions being arranged at both end of a balloon arrangement, arranging only one protrusion at one end of the balloon arrangement can restrain the stent at least axially. Preferably, the protrusion is, for example, inverted L-shaped so as to further restrain the stent radially.

In order to obtain balloon arrangements according to embodiments of the present invention, there are also provided in embodiments of the present invention methods of manufacturing such balloon arrangements, which may include the steps as detailed below.

Forming and shaping a protrusion at one end of a preform of the balloon arrangement through heating the preform in a molding chamber of a preforming mold and then filling a fluid in the mold, such that a molded structure of the balloon arrangement is obtained. Wherein, each of the preform and the molded structure is in a folded configuration, and a stent is crimped onto the molded structure so that it is located on the end where the protrusion is formed. Moreover, a height of the protrusion is at least higher than that of an inner surface of the crimped stent.

Since balloon arrangements with protrusions arranged at both end thereof operate in the same way as those with only one protrusion at one end, the following further detailed description will be given with protrusions being arranged at both ends of the balloon arrangement as an example for illustrating the manufacturing of various balloon arrangement versions.

At first, a method of manufacturing the balloon arrangement 10 as discussed above will be described as an example. The method may include the following steps 11 to 13.

In step 11, a preform 101 is provided in a folded configuration.

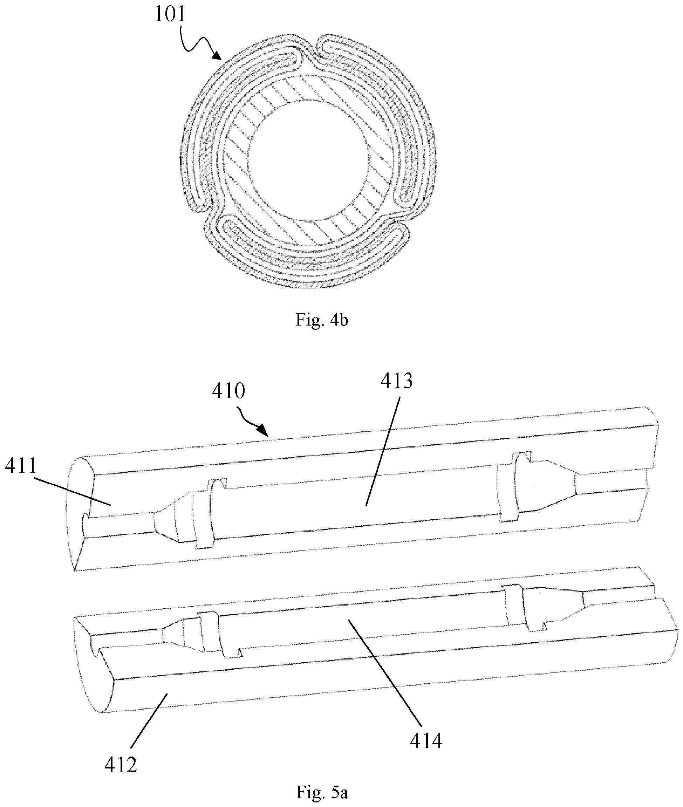

The preform 101 may have a structure as shown in Figs. 4a and 4b. As apparent from the figures, the preform 101 is in a folded configuration, which may be, but is not limited to, a tri-fold, single-fold, bi-fold or non-inflated balloon configuration. The preform 101 is made of a polymer, preferably polyamide.

In step 12, the preforming mold 410 is disposed over the preform 101 so that the preform 101 is housed within a molding chamber of the preforming mold 410, and the preforming mold 410 is then fixed. The molding chamber is matched in shape to the preform of the balloon arrangement 10.

As shown in Fig. 5a, an embodiment of the present invention is provided with a preforming mold 410, the preforming mold 410 preferably includes a first molding body 411 and a second molding body 412, the first molding body 411 defines a first molding cavity 413 and the second molding body 412 defines a second molding cavity 413. When the first molding body 411 is put together with the second molding body 412, the first molding cavity 413 and the second molding cavity 414 make up the molding chamber that is designed to have the same shape as the preform of the balloon arrangement 10. Preferably, in portions of the molding bodies that correspond to the protrusions, a number of small holes are arranged (preferably, uniformly) for facilitating venting of any gas from the molding cavities so that the balloon can better fit the molding cavities, allowing for better formation of the protrusions.

In step 13, the preform 101 is heated in the molding chamber of the preforming mold 410 and a compressed gas is filled therein, so that the protrusions 11 are formed and shaped at both ends of the preform 101. In this way, a molded structure of the balloon arrangement 10 is obtained.

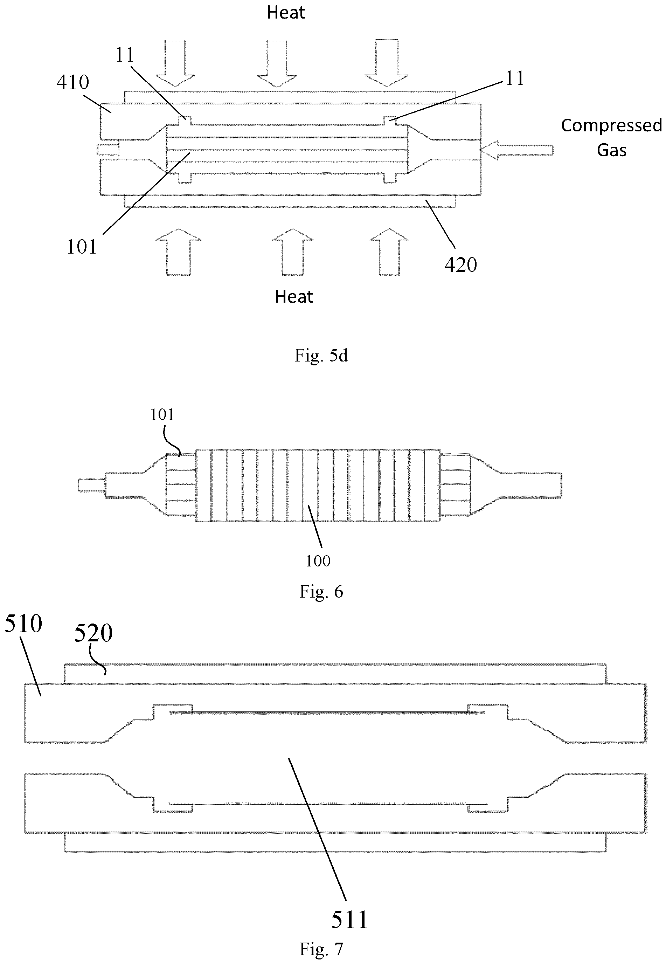

As shown in Fig. 5d, a heat source may be arranged outside the preforming mold 410 in order to overall heat the preforming mold 410, and the compressed gas may be injected into the preform 101 via a supply pipe so that the protrusions 11 are formed and shaped at both ends of the preform 101 in the molding chamber.

Further, after the molded structure of the balloon arrangement 10 has been obtained, a stent 100 may be crimped on the molded structure so that it is located between the protrusions 11 at the respective ends, as shown in Fig. 1b.

As for the balloon arrangement 20 as discussed above, since its method of use is similar to that of the balloon arrangement 10, i.e., an axial distance between the protrusions at its both ends that is greater than a length of the stent, reference can be made to the foregoing description of the manufacturing of the balloon arrangement 10 for details in the manufacturing of the balloon arrangement 20, and a repeated description thereof will be omitted.

Preferably, the heat source can be removed (to stop heating the preforming mold 410) after a duration of heating and venting, with the filling of the compressed gas into the preform being continued preferably until the mold has been cooled, in order to ensure a good formation result. Here, it should be recognized that after the molded structure is obtained, there is no compressed gas remaining within the preform 101. That is, the molded structure is in a non-inflated, contracted configuration. Additionally, during the formation of the protrusions 11, it may also be necessary to control the heating temperature and the pressure for supplying the compressed gas. In this embodiment, preferably, the heating temperature may range from 40 °C to 80 °C, and the pressure may range from 2.0 atm to 10.0 atm.

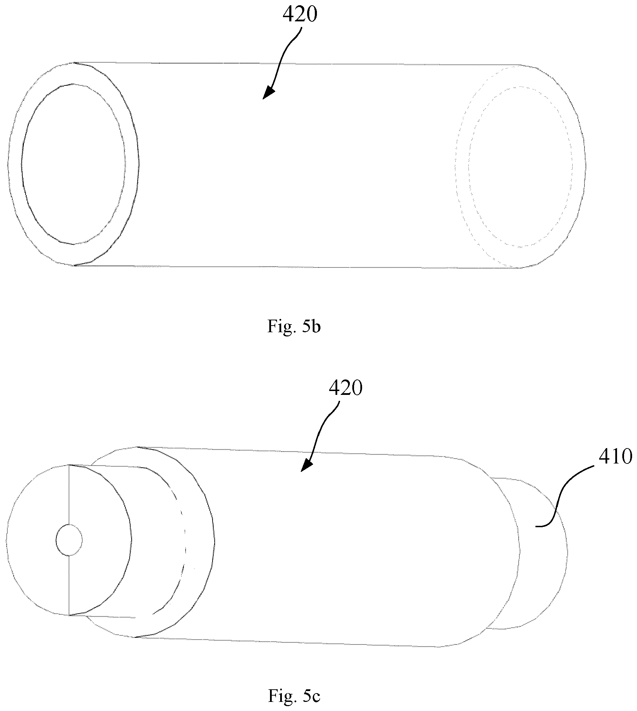

Next, with reference to Fig. 5b, in an embodiment of the present invention, there is also provided a fixture 420 for fixing the preforming mold 410. As shown in Fig. 5c, the fixture 420 is a hollow structure that is open at both ends and configured to be disposed over the closed preforming mold 410. However, the present invention is not so limited, because the fixture 420 may also fix the preforming mold 410 by clamping it, for example, using a multi-jaw chuck. Therefore, the present invention is not limited to any particular method of fixing the preforming mold 410 by the fixture 420.

Further, since the balloon arrangement 30 is so configured that a stent is loaded thereon while being wrapped by the balloon arrangement 30, the manufacturing of the balloon arrangement 30 differs from that of the balloon arrangements 10 and 20. In particular, a method for manufacturing the balloon arrangement 30 may include the following steps 21 to 24.

In step 21, a preform 101 in a folded configuration is provided, as shown in Figs. 4a and 4b. The folded configuration may be, but is not limited to, a tri-fold, single-fold, bi-fold or non-inflated balloon configuration.

In step 22, a stent 100 is crimped onto the preform 101, as shown in Fig. 6.

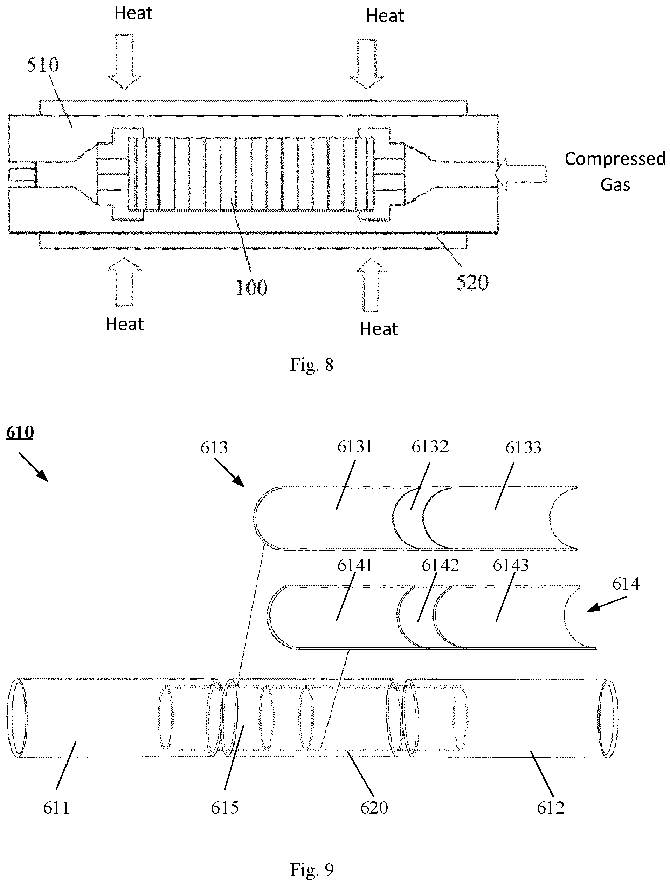

In step 23, a preforming mold 510 is disposed over the preform 101, on which the stent 100 is crimped, so that the preform 101 on which the stent 100 is crimped is housed within a molding chamber of the preforming mold 510, and the preforming mold 510 is then fixed, as shown in Fig. 8.

Here, referring to Fig. 7, in connection with Fig. 8, another preforming mold 510 provided in an embodiment of the present invention includes a molding chamber 511, the molding chamber 511 is matched in shape with the balloon arrangement 30. Preferably, the preforming mold 510 includes two molding bodies which, when closed, together define the molding chamber 511. In the closed configuration, the preforming mold 510 may be likewise fixed using a fixture 520. The fixture 520 may be structured similarly to the above-described fixture 420, and a repeated description thereof will be omitted.

In step 24, the preform 101 with the stent 100 crimped thereon is heated in the molding chamber 511 of the preforming mold 510 and a compressed gas is filled therein, so that protrusions 31 are formed and shaped at both ends of the preform 101. In this way, a molded structure of the balloon arrangement 30 is obtained.

As shown in Fig. 8, a heat source may be arranged outside the preforming mold 510 in order to mainly heat the portions of the preforming mold 510 that correspond to the protrusions 31 being formed, and the compressed gas may be introduced into the preform 101 to form the protrusions 31. Thus, differing from the above-discussed two balloon arrangements, the stent 100 is crimped onto the preform 101 before the molded structure of the balloon arrangement 30 is obtained. Since the stent 100 has been loaded on the molded structure, a subsequent step of crimping the stent is saved, making the manufacturing easier.

The method for manufacturing the balloon arrangement 30 is also applicable to the manufacturing of the above-discussed two balloon arrangements 10 and 20. That is, the stent is crimped onto the preform in the folded configuration before the protrusions are formed. Further, the present invention is not limited to any particular structure of the preforming mold, as long as the molding chamber is provided.

In other embodiments, another preforming mold 610 and a corresponding set process steps are provided for the balloon arrangement 30. Referring to Figs. 9, 10 and 11, the preforming mold 610 includes a confining cavity 615 that is matched in shape with the preform of the balloon arrangement 30. Preferably, the preforming mold 610 includes two confining molds and two shaping molds. The confining cavity 615 is defined together by the two confining molds when they are closed. The first confining mold 613 include a first flap 6131, a second flap 6132 and a third flap 6133, which are sequentially connected one to another, and the second confining mold 614 includes a fourth flap 6141, a fifth flap 6142 and a sixth flap 6143, which are sequentially connected one to another. In the closed configuration, the preforming mold 610 may be further fixed using a fixture 620. The fixture 620 may be structured similarly to the above-described fixture 420, and a repeated description thereof will be omitted.

The steps include steps 31 to 34, in which steps 31 and 32 are the same as steps 21 and 22, respectively.

In step 33, the first and second confining molds 613, 614 are disposed over the preform 101 with the stent 100 crimped thereon so that the two confining molds are closed in a seamless manner in which opposing ends of the stent are totally covered by respective ends of the confining molds, and a fixing sleeve 620 is then disposed over the confining molds so that the confining molds are tightly fitted against the stent.

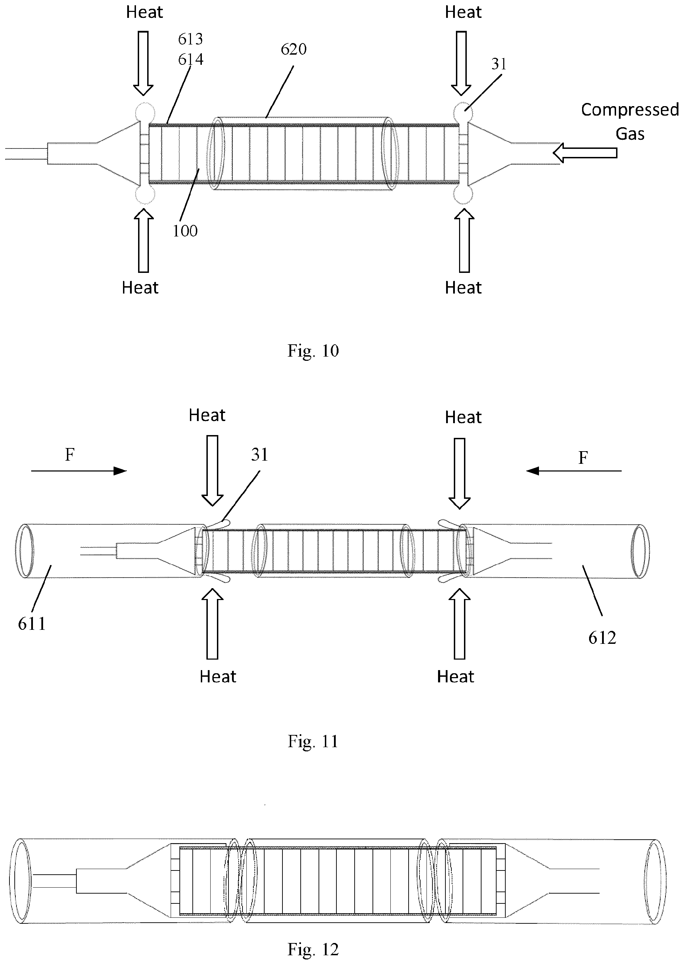

In step 34, a compressed gas is injected into the preform 101 that retains the crimped stent 100 thereon and is housed in the confining cavity, and balloon portions around opposing ends of the stent are heated so that the protrusions 31 are formed at the respective ends of the preform 101 (as shown in Fig. 10). The shaping molds 611 and 612 are then disposed over the protrusions, followed by reducing the pressure and concurrently causing the two shaping molds to move toward each other (as shown in Fig. 11). As a result, the balloon (i.e., the protrusions 31) deforms/moves from the ends of the stents to the inside/middle thereof, thus wrapping the stent and resulting in the formation of the molded structure of the balloon arrangement 30 (as shown in Figs. 12 and 3). After that, heating is stopped, and the molds are removed when the article has cooled off. The shaping molds define an inner diameter that is greater than the stent's diameter, and may terminate in the shape of flares that can facilitate the inward movement of the balloon. During the removal of the confining molds, the second and fifth flaps 6132 and 6142 may be removed first, and each remaining flap may be displaced toward the middle of the stent and taken away from the stent when it has moved away from the wrapped end portion of the stent.

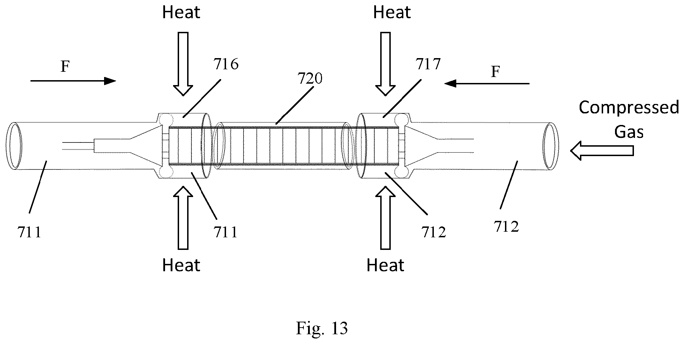

For the above molds, the present invention also provides another mold arrangement 710, which as shown in Fig. 13, consists of two shaping molds, two confining molds and a fixture. The shaping molds may be fixed using other means than as shown. The first and second confining molds function in the same way as those in the foregoing embodiment, while the first and second shaping molds 711, 712 are respectively disposed at both ends of the stent so as to form a first molding cavity 716 and a second molding cavity 717, respectively, each having a flare shape. The first molding cavity 716 has a first diameter and a second diameter, and the second molding cavity 717 has a third diameter and a fourth diameter. Both the second and third diameters are greater than a final circumferential diameter at the protrusions and are equal to a preset intermediate circumferential diameter at the protrusions. The first and fourth diameters are equal to the final circumferential diameter at the protrusions. Other structural details are the same as those of 610, and a repeated description thereof will be omitted.

A corresponding set of process steps includes steps 41 to 44, in which steps 41 and 42 are the same as steps 21 and 22, respectively.

In step 43, the first and second confining molds 713, 714 are disposed over the preform 101 with the stent 100 crimped thereon so that the two confining molds are closed in a seamless manner in which opposing ends of the stent are totally covered by respective ends of the confining molds, and a fixing sleeve 720 is then disposed over the confining molds so that the confining molds are tightly fitted against the stent. The first and second shaping molds 711, 712 are then disposed at the respective ends of the stent so that each portion of the folded balloon that extends beyond a respective end of the stent is aligned with a section where the corresponding mold transitions from the second diameter to the third diameter. As a result, the first and second molding cavities 716 and 717 are formed.

In step 44, a compressed gas is injected into the preform 101 that retains the crimped stent 100 thereon and is housed in the confining cavity, and balloon portions around opposing ends of the stent are heated so that the protrusions 31 are formed at the respective ends of the preform 101. Afterward, the pressure is reduced, and the two shaping molds are concurrently caused to move toward each other so that the balloon (i.e., the protrusions 31) deforms/moves from the ends of the stents to the inside/middle thereof, thus wrapping the stent and resulting in the formation of the molded structure of the balloon arrangement 30 (as shown in Fig. 3). After that, heating is stopped, and the molds are removed when the article has cooled off. With similarity to step 34, during the removal of the confining molds, the second and fifth flaps may be removed first, and each remaining flap may be displaced toward the middle of the stent and taken away from the stent when it has moved away from the wrapped end portion of the stent. Furthermore, in order to facilitate the removal, each shaping mold may be similarly made up of several portions.

Therefore, according to embodiments of the present invention, an existing folded balloon may be reshaped by expending its one or both ends into protrusion(s) in a preforming mold at a preset temperature and a predetermined fluid pressure. Here, as each protrusion is formed by the gas remaining in the folded balloon, it will not lead to a change in the wall thickness of the balloon. Therefore, when the balloon is inflated and overall expanded, the protrusion(s) will just disappear without impairing the balloon's performance. Loading and delivering a stent 100 with a balloon arrangement according to embodiments of the present invention, which is disposed at a distal end of a delivery catheter, can prevent the stent 100 from dislodging from the balloon arrangement when the distal end is being advanced through a more or less clotted, curved or tortuous blood vessel portion, thereby resulting in a reduction in surgical risk.

Additionally, in embodiments of the present invention, there is also provided a medical device including a stent and a balloon arrangement according to an embodiment of the present invention. The stent is crimped onto the balloon arrangement so that it is located on one end thereof where a protrusion is formed.

In the following, there are presented comparative data obtained from the embodiments of the present invention. First, data about dislodgement resistance of the preform 101 and the balloon arrangement 10 are summarized in Table 1.

The balloon arrangement was made of polyamide and the stent 100 was made of a cobalt-chromium alloy. The protrusion 11 was circumferentially continuous, the protrusion 11 had a total height of 150 µm, and the protrusion 11 was spaced from the stent by an axial clearance of 0.1mm. The dislodgement resistance was tested on a tensile tester. With the tensile tester clamping both a proximal end of the metal stent and a distal end of the balloon, the metal stent is pulled toward the distal of the balloon, and forces respectively required to displace the stent 0-5 mm and cause its complete dislodgement were recorded.

**Table 1 Dislodgement resistance of the preform 101 and balloon arrangement 10**

| Preform 101 | | | | |
|---|---|---|---|---|
| Displacement | First Group (N) | Second Group (N) | Third Group (N) | Average (N) |
| 0-5 mm | 3.06 | 3.57 | 3.21 | 3.28 |
| Complete Dislodgement | 4.65 | 5.66 | 7.15 | 5.82 |

| Balloon Arrangement 10 | | | | |
|---|---|---|---|---|
| Displacement | First Group (N) | Second Group (N) | Third Group (N) | Average (N) |
| 0-5mm | 5.42 | 5.29 | 5.22 | 5.31 |
| Complete Dislodgement | 9.62 | 8.46 | 10.08 | 9.39 |

As can be seen from Table 1, the balloon arrangement 10 according to this embodiment exhibited dislodgement resistance about 1.6 times that of the existing one. Therefore, the balloon arrangement 10 of the present invention can better prevent stent dislodgement.

Data about dislodgement resistance of the preform 101 and the balloon arrangement 30 are summarized in Table 2.

The balloon arrangement was made of polyamide and the stent 100 was made of a cobalt-chromium alloy. The protrusion 31 was circumferentially continuous, the protrusion 31 was wrapped an axial length of the stent 100 that was equal to 0.5 ring widths, and the protrusion 31 had a total height of 120 µm. The dislodgement resistance was tested on a tensile tester. With the tensile tester clamping both a proximal end of the metal stent and a distal end of the balloon, the metal stent is pulled toward the distal of the balloon, and forces respectively required to displace the stent 0-5 mm and cause its complete dislodgement were recorded.

**Table 2 Dislodgement resistance of the preform 101 and balloon arrangement 30**

| Preform 101 | | | | |
|---|---|---|---|---|
| Displacement | First Group (N) | Second Group (N) | Third Group (N) | Average (N) |
| 0-5mm | 3.06 | 3.29 | 3.11 | 3.15 |
| Complete Dislodgement | 4.8 | 5.26 | 5.09 | 5.05 |

| Balloon arrangement 30 | | | | |
|---|---|---|---|---|
| Displacement | First Group (N) | Second Group (N) | Third Group (N) | Average (N) |
| 0-5mm | 4.98 | 5.07 | 5.14 | 5.06 |
| Complete Dislodgement | 7.94 | 8.33 | 8.25 | 8.14 |

As can be seen from Table 2, the balloon arrangement 30 according to this embodiment exhibited dislodgement resistance that was 60% and 61% higher, compared to the existing one. Therefore, the balloon arrangement 30 of the present invention can better prevent stent dislodgement.

Burst pressure data of the preform 101 and the balloon arrangement 20 are summarized in Table 3.

The balloon arrangement was made of polyamide and the stent 100 was made of a cobalt-chromium alloy. The protrusion 21 had a total height of 130 µm and the protrusion 21 was spaced from the stent 100 by an axial clearance of 1.0 mm. In a 37°C water bath, the pressure was increased from 0 atm in increments of 1 atm and maintained for 10 s at each increment, until the balloon burst.

**Table 3 Burst pressures of the preform 101 and balloon arrangement 20**

| Preform 101 | | | | |
|---|---|---|---|---|
| No. | First Group | Second Group | Third Group | Average |
| Rated Burst Pressure | 22.09 | 22.04 | 23.35 | 22.5 |

| Balloon Arrangement 20 | | | | |
|---|---|---|---|---|
| No. | First Group | Second Group | Third Group | Average |
| Rated Burst Pressure | 23.02 | 23.06 | 22.05 | 22.7 |

As can be seen from Table 3, compared to the existing one, the balloon arrangement 20 of the present invention maintained comparable burst performance. Therefore, the preformation will not lower the balloon arrangement's performance during use, and the balloon arrangement maintains sufficient structural strength.

**Table 4 Delivery performance of a polymer stent system using the balloon arrangement 30**

| No. | Maximum Resistance Encountered during Delivery | Sampling Point (mm) |
|---|---|---|
| Balloon Arrangement 30 | 43.8 | 140.08 |
| Control | 46.4 | 139.76 |

As can be seen from Table 4, the addition of the protrusion had little impact on the stent delivery performance. This is because the material of the balloon is softer than that of the stent and wrapping the end of the stent with the balloon material can not only create a transition area that can effectively reduce damage to the blood vessel wall but can also prevent the stent end's bifurcation into a flare shape in the process of delivery.

In summary, in embodiments of the present invention, balloon arrangements are provided for loading and delivering stents, which are preferably polymer stents or metal stents, more preferably degradable stents.

The description presented above is merely that of a few preferred embodiments of the present invention without limiting the scope thereof in any sense. Any and all changes and modifications made by those of ordinary skill in the art based on the above teachings fall within the scope of the invention as long as included by the language of the appended claims.

## Claims

1. A method of manufacturing a medical device, comprising:
providing a preform (101) of a balloon arrangement (10, 20, 30) in a folded configuration;
crimping a stent (100) onto the preform (101);
disposing a preforming mold (410, 510, 610, 710) over the preform (101) on which the stent (100) is crimped so that the preform (101) on which the stent (100) is crimped is housed in a molding chamber (511, 716) of the preforming mold (410, 510, 610, 710); and
heating the preform (101) in the molding chamber (511, 716) and filling a fluid in the preform (101) so that a protrusion (11, 21, 31) is formed and shaped on the preform (101) in the molding chamber (511, 716), thereby obtaining a molded structure of the balloon arrangement (10, 20, 30), wherein:
the protrusion (21, 31) has a cross section of an inverted L shape along an axial direction of the molded structure.

2. The method of manufacturing a medical device of claim 1, wherein protrusions (11, 21, 31) are formed at both ends of the molded structure, and wherein the stent (100) is crimped on the molded structure and located between the protrusions (11, 21, 31) at both ends of the molded structure.

3. The method of manufacturing a medical device of claim 1, wherein during the obtainment of the molded structure, a surface of the protrusion(s) is fitted against an outer surface of the crimped stent (100) so that the protrusion(s) wraps the stent (100).

4. The method of manufacturing a medical device of claim 1 or 2, wherein after each protrusion (11, 21, 31) has been formed and shaped on the preform (101) in the molding chamber (511, 716), the heating of the preforming mold (410, 510, 610, 710) is stopped, with the filling of the fluid into the preform (101) being continued until the preforming mold (410, 510, 610, 710) has cooled off.

5. The method of manufacturing a medical device of claim 1, wherein the preform (101) is heated at a temperature of from 40 °C to 80 °C, and wherein the fluid is a compressed gas supplied at a pressure of from 2.0 atm to 10.0 atm.

6. A medical device comprising a stent (100) and a balloon arrangement (10, 20, 30) configured to release the stent (100) in an expanded configuration thereof, wherein the medical device is manufactured by the method as defined in any one of claims 1 to 5, wherein the balloon arrangement (10, 20, 30) comprises a molded structure with a protrusion (11, 21, 31), and wherein the stent (100) is crimped on the balloon arrangement (10, 20, 30) and located on a side where the protrusion (11, 21, 31) is formed, wherein:
the protrusion (21, 31) has a cross section of an inverted L shape along an axial direction of the molded structure.

7. The medical device of claim 6, wherein the protrusion (11, 21, 31) is circumferentially continuous, or wherein the protrusion (11, 21, 31) comprises a plurality of portions, the plurality of portions spaced apart from one another along a circumferential direction of the molded structure.

8. The medical device of claim 7, wherein an axial clearance between the protrusion (11, 21, 31) and the stent (100) is smaller than or equal to 5 mm.

9. The medical device of claim 6, wherein the protrusion (11, 21, 31) defines an opening that is open toward the molded structure and configured to accommodate an end portion of the stent (100).

10. The medical device of claim 9, wherein a surface of the protrusion (11, 21, 31) is fitted against an outer surface of the crimped stent (100) so that the protrusion (11, 21, 31) wraps at least part of the stent (100).

11. The medical device of claim 10, wherein 0 to 1.0 ring of the stent (100) in an axial direction is wrapped by the protrusion (11, 21, 31).

## Patentansprüche

1. Verfahren zum Herstellen einer medizinischen Vorrichtung, umfassend:
Bereitstellen einer Vorform (101) einer Ballonanordnung (10, 20, 30) in einer gefalteten Konfiguration;
Crimpen eines Stents (100) auf die Vorform (101);
Anordnen einer vorformenden Form (410, 510, 610, 710) über der Vorform (101), auf die der Stent (100) gecrimpt ist, so dass die Vorform (101), auf die der Stent (100) gecrimpt ist, in einer Formkammer (511, 716) der vorformenden Form (410, 510, 610, 710) untergebracht ist; und
Erhitzen der Vorform (101) in der Formkammer (511, 716) und Füllen eines Fluids in die Vorform (101), so dass ein Vorsprung (11, 21, 31) auf der Vorform (101) in der Formkammer (511, 716) gebildet und gestaltet wird, wodurch ein geformtes Gebilde der Ballonanordnung (10, 20, 30) erhalten wird, wobei:
der Vorsprung (21,31) einen Querschnitt einer umgekehrten L-Gestalt einer axialen Richtung des geformten Gebildes entlang aufweist.

2. Verfahren zum Herstellen einer medizinischen Vorrichtung nach Anspruch 1, wobei Vorsprünge (11, 21, 31) an beiden Enden des geformten Gebildes gebildet werden und wobei der Stent (100) auf das geformte Gebilde gecrimpt wird und sich zwischen den Vorsprüngen (11, 21, 31) an beiden Enden des geformten Gebildes befindet.

3. Verfahren zum Herstellen einer medizinischen Vorrichtung nach Anspruch 1, wobei, während des Erhaltens des geformten Gebildes, eine Oberfläche des Vorsprungs/der Vorsprünge gegen eine Außenfläche des gecrimpten Stents (100) so gepasst wird, dass der Vorsprung/die Vorsprünge den Stent (100) umwickelt/umwickeln.

4. Verfahren zum Herstellen einer medizinischen Vorrichtung nach Anspruch 1 oder 2, wobei, nachdem jeder Vorsprung (11, 21, 31) auf der Vorform (101) in der Formkammer (511, 716) gebildet und gestaltet worden ist, das Erhitzen der vorformenden Form (410, 510, 610, 710) gestoppt wird, wobei das Füllen des Fluids in die Vorform (101) fortgeführt wird, bis die vorformende Form (410, 510, 610, 710) abgekühlt ist.

5. Verfahren zum Herstellen einer medizinischen Vorrichtung nach Anspruch 1, wobei, die Vorform (101) bei einer Temperatur von 40 °C bis 80 °C erhitzt wird und wobei das Fluid ein komprimiertes Gas ist, das bei einem Druck von 2,0 atm bis 10,0 atm geliefert wird.

6. Medizinische Vorrichtung umfassend einen Stent (100) und eine Ballonanordnung (10, 20, 30), die konfiguriert ist, den Stent (100) in einer expandierten Konfiguration davon freizugeben, wobei die medizinische Vorrichtung durch das Verfahren wie in einem der Ansprüche 1 bis 5 definiert hergestellt wird, wobei die Ballonanordnung (10, 20, 30) ein geformtes Gebilde mit einem Vorsprung (11, 21, 31) umfasst und wobei der Stent (100) auf die Ballonanordnung (10, 20, 30) gecrimpt wird und sich auf einer Seite befindet, wo der Vorsprung (11, 21, 31) gebildet ist, wobei
der Vorsprung (21, 31) einen Querschnitt einer umgekehrten L-Gestalt einer axialen Richtung des geformten Gebildes entlang aufweist.

7. Medizinische Vorrichtung nach Anspruch 6, wobei der Vorsprung (11, 21, 31) zirkumferentiell kontinuierlich ist oder wobei der Vorsprung (11, 21, 31) eine Vielzahl von Abschnitten umfasst, wobei die Vielzahl von Abschnitten voneinander einer zirkumferentiellen Richtung des geformten Gebildes entlang beabstandet sind.

8. Medizinische Vorrichtung nach Anspruch 7, wobei ein axialer Abstand zwischen dem Vorsprung (11, 21, 31) und dem Stent (100) kleiner als oder gleich 5 mm ist.

9. Medizinische Vorrichtung nach Anspruch 6, wobei der Vorsprung (11, 21, 31) eine Öffnung definiert, die sich in Richtung der geformten Gebilde öffnet und konfiguriert ist, einen Endabschnitt des Stents (100) unterzubringen.

10. Medizinische Vorrichtung nach Anspruch 9, wobei eine Oberfläche des Vorsprungs (11, 21, 31) gegen eine Außenfläche des gecrimpten Stents (100) gepasst ist, so dass der Vorsprung (11, 21, 31) mindestens einen Teil des Stents (100) umwickelt.

11. Medizinische Vorrichtung nach Anspruch 10, wobei 0 bis 1,0 Ring des Stents (100) in einer axialen Richtung durch den Vorsprung (11, 21, 31) umwickelt ist.

## Revendications

1. Procédé de fabrication d'un dispositif médical, comprenant les étapes consistant à :
fournir une préforme (101) d'un agencement de ballonnet (10, 20, 30) dans une configuration pliée ;
sertir un stent (100) sur la préforme (101) ;
mettre en place un moule de préformage (410, 510, 610, 710) sur la préforme (101) sur laquelle le stent (100) est serti de sorte que la préforme (101) sur laquelle le stent (100) est serti est logée dans une chambre de moulage (511, 716) du moule de préformage (410, 510, 610, 710) ; et
chauffer la préforme (101) dans la chambre de moulage (511, 716) et remplir la préforme (101) d'un fluide de sorte qu'une protubérance (11, 21, 31) soit formée et mise en forme sur la préforme (101) dans la chambre de moulage (511, 716), moyennant quoi est obtenue une structure moulée de l'agencement de ballonnet (10, 20, 30), dans lequel :
la protubérance (21, 31) présente une section transversale en forme de L inversé le long d'une direction axiale de la structure moulée.

2. Procédé de fabrication d'un dispositif médical selon la revendication 1, dans lequel des protubérances (11, 21, 31) sont formées aux deux extrémités de la structure moulée, et dans lequel le stent (100) est serti sur la structure moulée et localisé entre les protubérances (11, 21, 31) aux deux extrémités de la structure moulée.

3. Procédé de fabrication d'un dispositif médical selon la revendication 1, dans lequel durant l'obtention de la structure moulée, une surface de la(des) protubérance(s) est ajustée contre une surface externe du stent (100) serti de sorte que la(les) protubérance(s) enveloppe(nt) le stent (100).

4. Procédé de fabrication d'un dispositif médical selon les revendication 1 ou 2, dans lequel après que chaque protubérance (11, 21, 31) a été formée et mise en forme sur la préforme (101) dans la chambre de moulage (511, 716), le chauffage du moule de préformage (410, 510, 610, 710) est stoppé, le remplissage du fluide dans la préforme (101) se poursuivant jusqu'à ce que le moule de préformage (410, 510, 610, 710) soit refroidi.

5. Procédé de fabrication d'un dispositif médical selon la revendication 1, dans lequel la préforme (101) est chauffée à une température de 40 °C à 80 °C, et dans lequel le fluide est un gaz comprimé alimenté à une pression de 2,0 atm à 10,0 atm.

6. Dispositif médical comprenant un stent (100) et un agencement de ballonnet (10, 20, 30) configuré pour libérer le stent (100) dans sa configuration déployée, dans lequel le dispositif médical est fabriqué par le procédé tel que défini selon l'une quelconque des revendications 1 à 5, dans lequel l'agencement de ballonnet (10, 20, 30) comprend une structure moulée avec une protubérance (11, 21, 31), et dans lequel le stent (100) est serti sur l'agencement de ballonnet (10, 20, 30) et localisé sur un côté où la protubérance (11, 21, 31) est formée, dans lequel :
la protubérance (21, 31) présente une section transversale en forme de L inversé le long d'une direction axiale de la structure moulée.

7. Dispositif médical selon la revendication 6, dans lequel la protubérance (11, 21, 31) est circonférentiellement continue, ou dans lequel la protubérance (11, 21, 31) comprend une pluralité de portions, la pluralité des portions étant espacées les unes des autres le long d'une direction circonférentielle de la structure moulée.

8. Dispositif médical selon la revendication 7, dans lequel un dégagement axial entre la protubérance (11, 21, 31) et le stent (100) est inférieur ou égal à 5 mm.

9. Dispositif médical selon la revendication 6, dans lequel la protubérance (11, 21, 31) définit une ouverture qui est ouverte vers la structure moulée et configurée pour loger une portion d'extrémité du stent (100).

10. Dispositif médical selon la revendication 9, dans lequel une surface de la protubérance (11, 21, 31) est ajustée contre une surface externe du stent (100) serti de sorte que la protubérance (11, 21, 31) enveloppe au moins une partie du stent (100).

11. Dispositif médical selon la revendication 10, dans lequel de 0 à 1,0 anneau du stent (100) dans une direction axiale est enveloppé par la protubérance (11, 21, 31).
